# EUROPEAN PATENT SPECIFICATION

(11) **EP 0 565 677 B1**
(45) Date of publication and mention of the grant of the patent: **21.08.1996**
(21) Application number: 92922391.5
(22) Date of filing: 28.10.1992
(51) Int. Cl.: G01F 3/00, A61M 5/168

(54) **OPTICAL FLOW DETECTOR**
Optischer Durchflussmesser
DETECTEUR OPTIQUE DE DEBIT

(30) Priority: 05.11.1991 IT TO910083
(43) Date of publication of application: 20.10.1993
(73) Proprietor: HOSPAL LTD., CH-4008 Basel (CH)
(72) Inventor: PAOLINI, Francesco, I-87100 Cosenza (IT); PARALUPPI, Marco, I-41036 Medolla (IT); VINCI, Luca, I-46025 Poggio Rusco (IT)
(86) International application number: EP9202457
(87) International publication number: WO9309406

(56) References cited:
- EP-A- 0 229 354
- WO-A-86/03597
- DE-A- 2 920 693
- US-A- 3 609 379
- US-A- 4 314 484

## Description

The present invention relates to a liquid flow detector of the type associated with a drip chamber formed by a transparent cup-shaped container into which there flows an inlet tube provided at its end with a nozzle disposed in the upper portion of the container. The liquid collected in the container flows from the base of the container via an outlet tube under the action of a peristaltic pump disposed in the outlet tube. Detectors of known type are applied to this container and detect the flow of liquid by means of optoelectronic sensors which comprise a photoemitter and a photodetector defining an optical path which intersects the flow of liquid.

Such detectors corresponding to the preamble of Claim 1 are known from DE-A-2 920 693 and US-A-4 314 484.

Flow detectors of known type also comprise an electronic unit which receives as input the signal supplied by the photodetector in order to ascertain whether the liquid is flowing into the container.

Moreover, detectors of known type are separated into two classes depending on the characteristics of the liquid flow (continuous or in droplets).

In the first class of detectors of digital type, the electronic unit counts the number of pulses of the input signal (caused by the droplets falling from the nozzle) in order to ascertain the quantity of liquid flowing into the container.

This first class of detector is not able to monitor the flow of liquid when it is continuous as the pulses of the input signal are missing.

In the second class of detectors of analog type, the electronic unit detects the amplitude of the input signal which is a function of the quantity of liquid falling from the nozzle and compares it with a reference level in order to measure the flow of this liquid.

This second class of detector is highly influenced by the characteristics of the container and the condition of the sensor and provides an incorrect reading when the container has yellowed because of wear or age. The presence of vapours in the container may also have an adverse effect on the measurement.

The object of the present invention is to provide a flow detector which resolves the drawbacks of known detectors.

This object is achieved by the present invention as defined in Claim 1.

The invention is described with reference to the accompanying drawings, in which:
Fig. 1 shows, in outline, a flow sensor comprising a unit and a probe associated with a drip chamber;
Fig. 2 is a first cross-section through the probe of Fig. 1;
Fig. 3 is a second cross-section through the probe of Fig. 1;

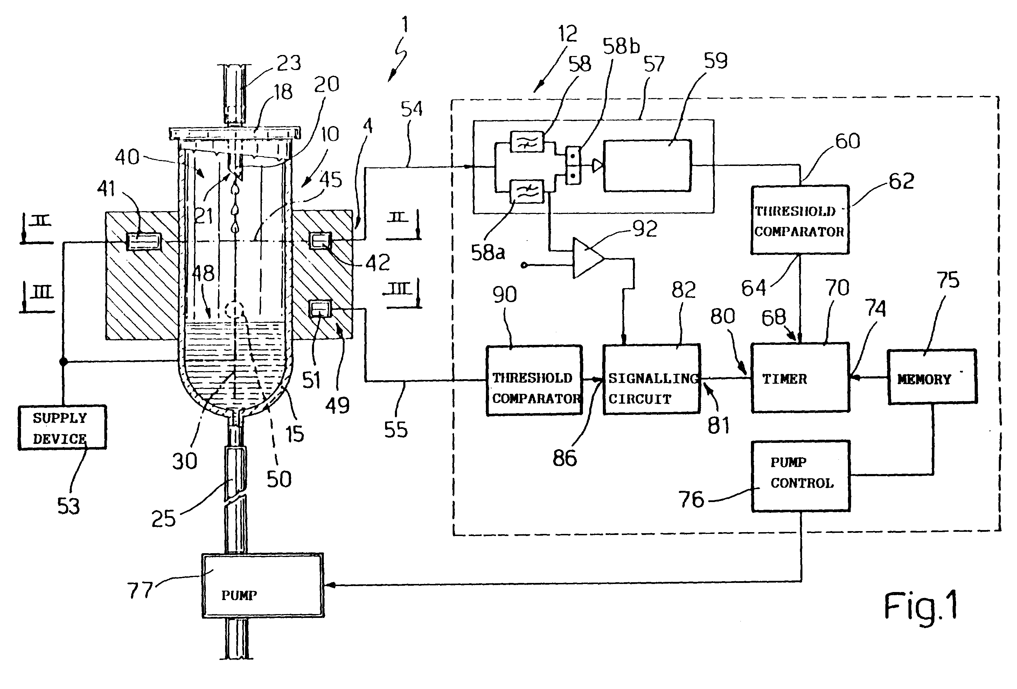

In Fig. 1, a flow detector is shown overall by 1 and comprises a probe 4 which is operationally associated with a drip chamber 10 into which a liquid flows and an electronic unit 12.

The drip chamber 10 is in particular formed by a tubular cup-shaped container 15 which is made from transparent plastic and is closed at the top by a plane stopper 18 traversed by a nozzle 20 provided with an inclined end edge 21 contained within the drip chamber 10. The nozzle 20 communicates with an inlet tube 23 within which the liquid flows.

The container 15 is also connected at the bottom to an outlet tube 25 which extends from a base wall of the container 15 and is connected to a pump 77.

The container 15 is disposed vertically with respect to a horizontal plane and the liquid discharged from the nozzle 20 drops along a vertical path 30 and is collected in a base portion of the container 15.

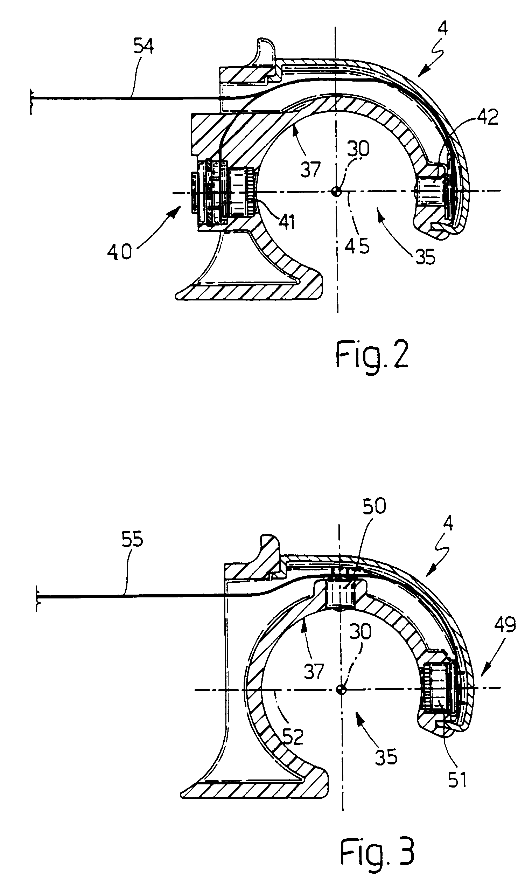

The probe 4 (Figs. 2 and 3) has a groove 35 which is limited by a cylindrical wall portion 37 and houses a central portion of the container 15 which is disposed in contact with the wall 37.

The probe 4 houses a first optoelectronic transducer 40 comprising a photoemitter 41 (for instance an LED diode) and a photodetector 42 (for instance a phototransistor) disposed on opposite sides of the wall 37 so as to define an optical path 45 passing through the container 15 and intersecting the path 30.

The probe 4 houses a second optoelectronic transducer 49 disposed below the transducer 40 at a distance from the base of the container such that, in normal operating conditions of the liquid flow system (correct flow velocity), the free surface 48 of the liquid housed in the lower portion of the container 15 is disposed below the transducer 49. The transducer 49 comprises a photoemitter 50 (for instance an LED diode) and a photodetector 51 (for instance a phototransistor) disposed perpendicular to one another. Consequently, in normal conditions, the rays generated by the photoemitter 50 are propagated along a cone of light with an opening angle such that they strike the photodetector 51; when, in contrast, the liquid level in the container 15 is high and reaches the transducer 49, the attenuation of the light by the liquid and the restriction of the light cone mean that the light rays generated by the photoemitter 50 do not reach the photodetector 51 which then generates a corresponding electrical signal.

The transducers 40 and 50 are supplied by a supply circuit 53 and are connected to the unit 12 by respective lines 54 and 55.

The unit 12 comprises a unit 57 which receives as input the signal from the photodetector 42 and generates, in a manner which will be explained in detail below, a control signal representative of the variability over time of the signal from the photodetector 42. This control signal is supplied to an input 60 of a comparator circuit 62 which has an output 64 connected to a first input 68 of a timer circuit 70.

The circuit 70 has a second input 74 connected to a memory 75 coupled to a pump control circuit 76 which is associated with the pump 77 of a fluid circulation plant (not shown) generally of the roller type.

The circuit 70 also has an output 80 which is connected to a first input 81 of a signalling circuit 82 having a second input 86 connected to a threshold comparator circuit 90.

The threshold comparator circuit 90 receives the signal generated by the photodetector 51 as input.

In use, the liquid from the tube 23 falls under the effect of gravity from the nozzle 20 along the path 30, passing through the optical path 45, and is collected on the base of the container 15. In this way, the signal generated by the photoemitter 41 is modulated by the liquid flowing from the nozzle 20 and the signal obtained by the photodetector 42 is variable over time as a function of the flow of fluid along the path 30.

The signal generated by the photoemitter 42 is variable over time only in the presence of a flow of liquid, since if the liquid falls in the form of droplets this signal has a pulse component due to the interruption of the optical path 45 caused by the droplets and if the liquid falls continuously this signal has a variable component over time due, on the one hand, to small momentaneous variations in flow because of the shape of the edge 21 and the surface tension of the liquid and, on the other hand, to the turbulence of the liquid falling from the nozzle 20, with the result that the index of refraction of the liquid varies in time and space.

The unit 57 generates a control signal which is proportional to the variability of the input signal.

For this purpose, the unit 57 comprises a high-pass filter 58, a low-pass filter 58a and a divider circuit 58b connected at its output with the input of a block 59. The filters 58a and 58 in particular have respective inputs connected with the line 54 and outputs connected with the divider circuit 58b so that the signal from the filter 58 is divided by the signal from the filter 58a and the signal produced by this division is supplied to the circuit 59.

In practice, the filter 58 eliminates the continuous component of the signal received by the unit 57 so as to keep solely the alternating component due to the pulses generated by the droplets or by the variations of flow and index of refraction in the case of a continuous flow.

This alternating component is then divided by the mean value of the signal received as output from the filter 58a so as to carry out a "standardization" of this alternating component; this operation is carried out in order to make the signal at the input of the circuit 59 independent of the amplitude of the signal generated by the photoemitter 42, preventing, for example, variations of the signal level due to the ageing of the photoemitter or the yellowing of the walls of the container 15 from affecting the operation of the unit 57.

The standardized alternating component output from the block 58b is then supplied to the block 59 which, for instance, calculates its mean quadratic value in order to eliminate disturbances and reduce the incidence of errors, and is then supplied to the circuit 62 in order to generate a flow absence signal when the control signal drops below a predetermined reference level.

The reduced size of the control signal is representative of an absence of flow along the path 30.

The flow absence signal is then supplied to the circuit 70 which processes it in order to correlate it with the flow velocity of the liquid determined by the velocity of the pump 77.

The circuit 70 in particular checks whether the flow absence signal is present continuously in a comparison time interval T, whose duration is smaller in the case of a high velocity of the pump 77 (and therefore a high liquid flow) or greater in the opposite case, and activates the circuit 82 if the result of this comparison is positive.

This check is carried out since the variability of the signal input to the unit 57 is, to some extent, correlated with the flow velocity of the liquid with the result, in the case of a discontinuous flow of droplets (with a few droplets per minute), that the flow absence signal may be generated because in this case the liquid flow is zero in the time intervals between two successive droplets.

Consequently, if the pump is operating at a low number of revolutions corresponding to a flow of liquid in droplet form, the flow absence signal may be present in the time intervals between the droplets.

The flow absence signal is therefore monitored over a broad time interval since the circuit 82 is actuated only if the flow absence signal is present throughout the time interval showing that no droplet has fallen during the whole of the time T.

If the pump 77 is operating at a high number of revolutions corresponding to a high liquid flow, the absence of the control signal shows a malfunction as the liquid flow, which should be high, is in practice zero.

This situation must be detected immediately because of the risks entailed, with the result that in this case the time interval during which the flow absence signal is generated by the comparator 62 is small. The time interval T is preferably selected from three different values, stored in the memory 75, corresponding to three different velocity intervals of the pump 77.

The flow absence signal which may have been generated by the timer 70 is then supplied to the signalling circuit 82 which generates a corresponding acoustic and/or optical signal to warn the operator of the absence of flow.

The unit 12 further comprises a comparator circuit 92 which has a first input connected to the output of the block 58a, a second input disposed at a reference potential close to zero and an output connected to the circuit 82.

The circuit 92 actuates the circuit 82 when its first input is at a voltage close to zero showing the absence of an input signal in the block 57 (due, for instance, to a malfunction of the photodetector 42).

The circuit 92 further prevents, in the absence of an input signal to the block 57, the divider block 58b from carrying out inadmissible operations, for instance the division of zero by zero whose result would be completely without significance.

The transducer 49 carries out the auxiliary function of monitoring the level of the liquid contained in the container 15 in order to disable the signalling circuit 82 when the free surface 48 of the liquid has reached the transducer 49. In this case, the liquid, because of disturbances due for instance to the pump 77, may interfere with the optical path 45 making detection by the transducer 40 impossible or unreliable.

If the liquid contained in the container 15 interferes with the optical path 52, the light generated by the photoemitter 50 no longer reaches the photodetector 51 which consequently does not transmit any output signal. The comparator circuit 90 which compares the output of the photodetector 51 with a reference level close to zero consequently detects this absence of signal and supplies a corresponding overflow signal to the alarm signalling circuit 82. This circuit 82 consequently disregards any alarm signal generated by the timer 70 and generates a malfunction signal.

It is clear from the above that the flow detector of the present invention resolves the drawbacks of known detectors. This detector is not sensitive to the level of the signal supplied by the photodetector but is sensitive to the variation of this signal over time; this prevents errors due to the variation of the signal level (due to the yellowing of the container or the presence of vapours).

The flow detector also makes it possible to control the flow of blood in various operating conditions (flow in the form of droplets or as a continuous flow) as a function of the fluid flow detected by measuring the velocity of the pump.

It is also clear that modifications and variants may be made to the present invention without departing from the scope of protection of the invention.

The unit 57, for instance, could comprise blocks differing from those described and could process the signal generated by the photodetector 42 in a different way. The block 59, for instance, could be replaced by a block adapted to calculate the absolute value of the standardized alternating component in order to eliminate disturbances and reduce the incidence of errors.

The transducer 49 could be of a type differing from that described, for instance comprising an ultrasound sensor.

### KEY TO THE DRAWINGS

### Fig. 1

- 53: Supply device
- 77: Pump
- 62: Threshold comparator
- 70: Timer
- 75: Memory
- 76: Pump control
- 82: Signalling circuit
- 90: Threshold comparator

## Claims

1. Liquid flow detector for monitoring a continuous as well as a discontinuous flow of liquid comprising :
- a container (10) having a vertical axis along which to flow a liquid ;
- a light emitter (41) ;
- a light detector (42) for generating a signal representative of a light received from the light emitter (41), the light emitter (41) and the light detector (42) being disposed with respect to the container (10) so as to define between them an optical path intersecting the vertical axis ;
- means (57) for processing the signal generated by the light detector (42) and for generating a control signal representative of the variation over time of the signal from the detector (42) ; and
- means (62) for generating a flow absence signal when the control signal drops below a predetermined level ; characterized in that it comprises :
- means for correlating (70, 75) the flow absence signal with a variable flow velocity of a liquid circulated by a pump (77) in a circuit connected to the container (10).

2. Liquid flow detector according to claim 1, characterized in that the correlating means (70, 75) comprises :
- means for storing (75) a plurality of preset comparison time intervals T ;
- means for selecting a specific comparison time interval T with respect to an actual velocity of the pump ;
- means for checking whether the flow absence signal is present continuously in the comparison time interval T.

3. Liquid flow detector according to claim 2, characterized in that it further comprises means (82) for signaling when the flow absence signal is present continuously in a comparison time interval T.

4. Liquid flow detector according to one of the claims 1 to 3, characterized in that the means (57) for processing the signal generated by the light detector (42) and for generating a control signal representative of the variability over time of the signal from the detector comprises :
- a high pass (58) filter for receiving the signal from the detector (42) and for generating a first signal ;
- a low pass filter (58a) for receiving the signal from the detector (42) and for generating a second signal ;
- dividing means (58b) for dividing the first signal by the second signal and generating a signal independent of an amplitude of the signal from the detector (42) ;
- means (59) for eliminating disturbances from the signal from the dividing means.

5. Liquid flow detector according to claim 4, characterized in that it further comprises means (92) for comparing the signal generated by the low pass filter (58a) to a reference potential close to zero.

6. Liquid flow detector according to claim 4 or 5, characterized in that the means (59) for eliminating disturbances from the signal from the dividing means comprises means for calculating the mean quadratic value of the signal from the dividing means.

7. Liquid flow detector according to claim 4 or 5, wherein the means for eliminating disturbances from the signal from the dividing means (58b) comprises means for calculating the absolute value of the signal from the dividing means (58b).

8. Liquid flow detector according to one of the claims 2 to 7, wherein T has a duration which is inversely proportional to the flow velocity of the liquid in the circuit connected to the container.

## Patentansprüche

1. Flüssigkeitsdurchflußdetektor zum Überwachen sowohl eines kontinuierlichen als auch eines diskontinuierlichen Durchflusses von Flüssigkeit, mit:
- einem Behälter (10) mit einer vertikalen Achse, entlang der eine Flüssigkeit strömen soll;
- einem Lichtemitter (41);
- einem Lichtdetektor (42) zum Erzeugen eines Signals, das ein von dem Lichtemitter (41) empfangenes Licht darstellt, wobei der Lichtemitter (41) und der Lichtdetektor (42) bezüglich des Behälters (10) derart angeordnet sind, daß sie zwischen sich einen die vertikale Achse schneidenden optischen Weg definieren;
- einem Mittel (57) zum Verarbeiten des von dem Lichtdetektor (42) erzeugten Signals und zum Erzeugen eines Steuersignals, das die zeitliche Veränderung des Signals vom Detektor (42) darstellt; und
- einem Mittel (62) zum Erzeugen eines Strömungsabwesenheitssignals, wenn das Steuersignal unter einen vorbestimmten Pegel fällt;
dadurch gekennzeichnet, daß er ein Mittel zum Inbeziehungsetzen (70, 75) des Strömungsabwesenheitssignals mit einer veränderlichen Durchflußgeschwindigkeit einer von einer Pumpe (77) in einem mit dem Behälter (10) verbundenen Kreislauf umgewälzten Flüssigkeit umfaßt.

2. Flüssigkeitsdurchflußdetektor nach Anspruch 1, dadurch gekennzeichnet, daß das Bezugsmittel (70, 75) folgendes umfaßt:
- ein Mittel zum Speichern (75) mehrerer voreingestellter Vergleichszeitintervalle T;
- ein Mittel zum Auswählen eines bestimmten Vergleichszeitintervalls T bezüglich einer lstgeschwindigkeit der Pumpe;
- ein Mittel zum Prüfen, ob das Strömungsabwesenheitssignal in dem Vergleichszeitintervall T kontinuierlich vorliegt.

3. Flüssigkeitsdurchflußdetektor nach Anspruch 2, dadurch gekennzeichnet, daß er weiterhin ein Mittel (82) zur Signalgebung, wenn das Strömungs-abwesenheitssignal in einem Vergleichszeitintervall T kontinuierlich vorliegt, umfaßt.

4. Flüssigkeitsdurchflußdetektor nach einem der Ansprüche 1 bis 3, dadurch gekennzeichnet, daß das Mittel (57) zum Verarbeiten des vom Lichtdetektor (42) erzeugten Signals und zum Erzeugen eines Steuersignals, das die zeitliche Veränderlichkeit des Signals vom Detektor darstellt, folgendes umfaßt:
- ein Hochpaßfilter (58) zum Empfangen des Signals vom Detektor (42) und zum Erzeugen eines ersten Signals;
- ein Tiefpaßfilter (58a) zum Empfangen des Signals vom Detektor (42) und zum Erzeugen eines zweiten Signals;
- ein teilendes Mittel (58b) zum Teilen des ersten Signals durch das zweite Signal und Erzeugen eines von einer Amplitude des Signals vom Detektor (42) unabhängigen Signals;
- ein Mittel (59) zum Entfernen von Störungen aus dem Signal von dem teilenden Mittel.

5. Flüssigkeitsdurchflußdetektor nach Anspruch 4, dadurch gekennzeichnet, daß er weiterhin ein Mittel (92) zum Vergleichen des von dem Tiefpaßfilter (58a) erzeugten Signals mit einem Bezugspotential nahe Null umfaßt.

6. Flüssigkeitsdurchflußdetektor nach Anspruch 4 oder 5, dadurch gekennzeichnet, daß das Mittel (59) zum Entfernen von Störungen aus dem Signal von dem teilenden Mittel ein Mittel zum Berechnen des mittleren quadratischen Werts des Signals aus dem teilenden Mittel umfaßt.

7. Flüssigkeitsdurchflußdetektor nach Anspruch 4 oder 5, wobei das Mittel zum Entfernen von Störungen aus dem Signal von dem teilenden Mittel (58b) ein Mittel zum Berechnen des Absolutwerts des Signals von dem teilenden Mittel (58b) umfaßt.

8. Flüssigkeitsdurchflußdetektor nach einem der Ansprüche 2 bis 7, wobei T eine Dauer aufweist, die umgekehrt proportional zu der Durchflußgeschwindigkeit der Flüssigkeit in dem mit dem Behälter verbundenen Kreislauf ist.

## Revendications

1. Détecteur d'écoulement de liquide pour contrôler un écoulement de liquide continu aussi bien qu'un écoulement de liquide discontinu comprenant :
- un conteneur (10) ayant un axe vertical le long duquel doit s'écouler un liquide ;
- un émetteur de lumière (41) ;
- un détecteur de lumière (42) pour engendrer un signal représentatif d'une lumière reçue de l'émetteur de lumière (41), l'émetteur de lumière (41) et le détecteur de lumière (42) étant disposés par rapport au conteneur (10) de façon à définir entre eux un trajet optique interceptant l'axe vertical ;
- des moyens (57) pour traiter le signal engendré par le détecteur de lumière (42) et pour engendrer un signal de commande représentatif de la variation dans le temps du signal provenant du détecteur (42) ; et
- des moyens (62) pour engendrer un signal d'absence d'écoulement lorsque le signal de commande décroît au-dessous d'un niveau prédéterminé ;
caractérisé en ce qu'il comporte des moyens (70, 75) pour corréler le signal d'absence d'écoulement à une vitesse d'écoulement variable d'un liquide mis en circulation par une pompe (77) disposée sur un circuit connecté au conteneur (10).

2. Détecteur d'écoulement de liquide selon la revendication 1 caractérisé en ce que les moyens de corrélation (70, 75) comprennent:
- des moyens (75) pour stocker une pluralité d'intervalles de temps de comparaison T prédéterminés ;
- des moyens pour sélectionner un intervalle de temps de comparaison T spécifique en fonction de la vitesse réelle de la pompe;
- des moyens pour vérifier si le signal d'absence d'écoulement est présent de façon continue pendant l'intervalle de temps de comparaison T.

3. Détecteur d'écoulement de liquide selon la revendication 2, caractérisé en ce qu'il comprend en outre des moyens (82) pour signaler quand le signal d'absence de liquide est présent de façon continue dans un intervalle de temps de comparaison T.

4. Détecteur d'écoulement de liquide selon une des revendications 1 à 3, caractérisé en ce que les moyens (57) pour traiter le signal engendré par le détecteur de lumière (42) et pour engendrer un signal de commande représentatif de la variation dans le temps du signal provenant du détecteur comprennent :
- un filtre passe-haut (58) pour recevoir le signal provenant du détecteur (42) et pour engendrer un premier signal ;
- un filtre passe-bas (58a) pour recevoir le signal (42) provenant du détecteur (42) et pour engendrer un second signal ;
- des moyens de division (58b) pour diviser le premier signal par le second signal et pour engendrer un signal indépendant de l'amplitude du signal provenant du détecteur (42) ;
- des moyens (59) pour éliminer des perturbations du signal provenant des moyens de division.

5. Détecteur d'écoulement de liquide selon la revendication 4, caractérisé en ce qu'il comprend en outre des moyens (92) pour comparer le signal engendré par le filtre passe-bas (58a) à un potentiel de référence proche de zéro.

6. Détecteur d'écoulement de liquide selon la revendication 4 ou 5, caractérisé en ce que les moyens (59) pour éliminer des perturbations du signal provenant des moyens de division comprennent des moyens pour calculer la valeur quadratique moyenne du signal provenant des moyens de division.

7. Détecteur d'écoulement de liquide selon la revendication 4 ou 5, dans lequel les moyens pour éliminer des perturbations du signal provenant des moyens de division (58b) comprennent des moyens pour calculer la valeur absolue du signal provenant des moyens de division (58b).

8. Détecteur d'écoulement de liquide selon l'une des revendications 2 à 7, dans lequel T a une durée qui est inversement proportionnelle à la vitesse d'écoulement du liquide dans le circuit connecté au conteneur.
